(19) Europäisches Patentamt

European Patent Office

Office européen des brevets

(11) **EP 4 331 469 B1**

(12) **EUROPÄISCHE PATENTSCHRIFT**

(45) Veröffentlichungstag und Bekanntmachung des Hinweises auf die Patenterteilung:
**01.10.2025  Patentblatt 2025/40**

(21) Anmeldenummer: **22192915.1**

(22) Anmeldetag: **30.08.2022**

(51) Internationale Patentklassifikation (IPC):
**A61B 3/103** $^{(2006.01)}$     **A61B 3/04** $^{(2006.01)}$

(52) Gemeinsame Patentklassifikation (CPC):
**A61B 3/103; A61B 3/04**

(54) **VORRICHTUNG ZUM PRÜFEN DES SEHENS DURCH EINE INTRAOKULARLINSE**

DEVICE FOR TESTING VISION BY MEANS OF AN INTRAOCULAR LENS

DISPOSITIF PERMETTANT DE CONTRÔLER LA VISION AU MOYEN D'UNE LENTILLE INTRAOCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.03.2024  Patentblatt 2024/10**

(73) Patentinhaber:
• **ACMIT Gmbh**
  **2700 Wiener Neustadt (AT)**
• **QMP Holding GmbH**
  **68163 Mannheim (DE)**

(72) Erfinder:
• **BREZNA, Wolfgang**
  **2700 Wiener Neustadt (AT)**
• **DRAGOSTINOFF, Nikolaus**
  **1230 Wien (AT)**
• **KORNFELD, Martin**
  **1100 Wien (AT)**
• **LA SCHIAZZA, Olivier**
  **69120 Heidelberg (DE)**

• **LUX, Kirsten**
  **2340 Mödling (AT)**
• **PLANK, Nicole**
  **7212 Forchtenstein (AT)**

(74) Vertreter: **Neuhold, Alfred**
  **IPrime Rentsch Kaelin AG**
  **Hirschengraben 1**
  **8001 Zürich (CH)**

(56) Entgegenhaltungen:
DE-A1- 102015 218 328     DE-A1- 3 118 561
JP-A- 2012 068 551     US-A- 5 159 496
US-A1- 2011 080 562     US-A1- 2019 004 335

• NORRBY ET AL: "Sources of error in intraocular lens power calculation", JOURNAL CATARACT AND REFRACTIVE SURGERY, SURGERY, FAIRFAX, VA, US, vol. 34, no. 3, 23 February 2008 (2008-02-23), pages 368 - 376, XP022496457, ISSN: 0886-3350, DOI: 10.1016/ J.JCRS.2007.10.031

EP 4 331 469 B1

**Beschreibung**

Die vorliegende Erfindung betrifft eine Vorrichtung zum Prüfen des Sehens durch eine Intraokularlinse, mit einem Träger, der in einer zentralen Sichtachse von distal zu proximal aufeinanderfolgend eine Cornea-Optik, eine Halterung für die Intraokularlinse und eine Okular-Optik lagert. Die Schrift JP 2012 068551 A zeigt eine solche Vorrichtung gemäß der Präambel von Anspruch 1.

Die Implantation einer phaken, aphaken oder pseudophaken Intraokularlinse ("Intraocular lens", IOL) im Auge eines Patienten ist ein überaus kritischer Prozess. Die IOL muss bestmöglich an die individuellen Erfordernisse des Patienten angepasst sein, da jeder spätere Austausch ein unnötiges Operationsrisiko in sich birgt. Daher ist es notwendig und wünschenswert, die Sicht des Patienten durch eine zu implantierende IOL bzw. deren optische Eigenschaften bereits vor einer Implantation gründlich zu überprüfen, um einen Austausch in vivo unter allen Umständen zu vermeiden.

Herkömmlicherweise werden dazu entweder Computersimulationen oder optische Prüfvorrichtungen, in welche die zu implantierende IOL eingesetzt wird, verwendet. Mittels Computersimulation anhand eines Auges mit durchschnittlicher Anatomie erstellte Bilder, die dem Patienten gezeigt werden, geben jedoch stets nur sehr allgemeine Eigenschaften einer IOL wieder. Mit Simulationen ist es daher unmöglich, den subjektiven Seheindruck des Patienten durch die für die Implantation ausgewählte IOL zuverlässig vorherzusagen. Darüberhinaus ist es besonders schwierig, unterschiedliche Sehsituationen zuverlässig zu simulieren, z.B. verschiedene Lichtverhältnisse und Sichtweiten wie die Fernsicht beim Betrachten einer Landschaft und die Nahsicht beim Lesen eines Buches.

Optische Prüfvorrichtungen eignen sich besser zur Überprüfung des subjektiven Seheindrucks in unterschiedlichen Situationen. Die derzeit bekannten Prüfvorrichtungen sind jedoch im Sichtfeld stark begrenzt und damit nicht imstande, einen großen Bereich an Sichtwinkeln des Patienten zu überprüfen.

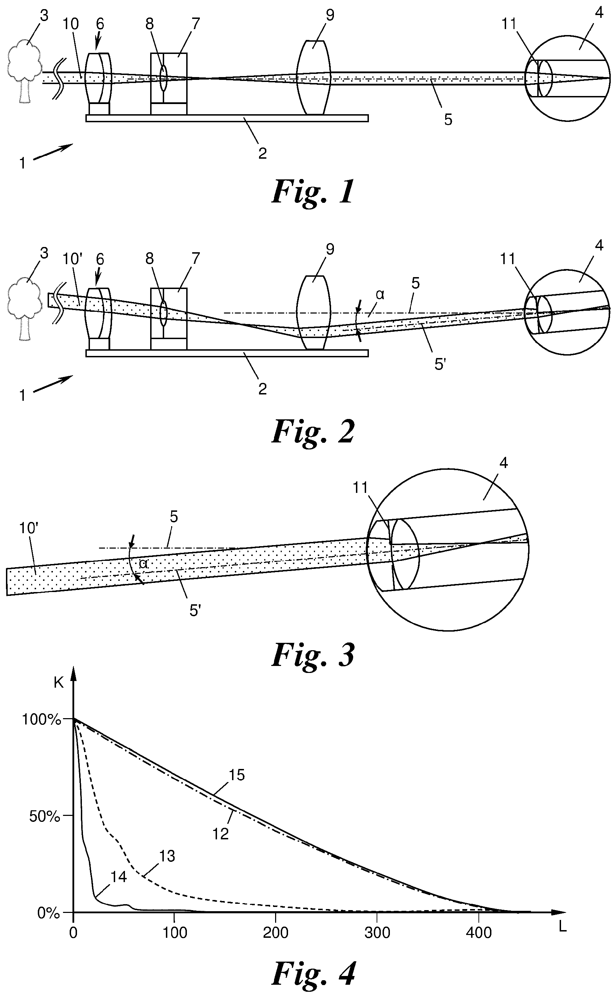

Die Fig. 1 bis 3 zeigen eine solche herkömmliche optische Prüfvorrichtung 1 mit einem Träger 2, der von distal (nahe zur betrachteten Umgebung 3) bis proximal (nahe zum Auge 4 des Patienten) entlang einer zentralen Sichtachse 5 eine Cornea-Optik 6 (auch Corneamodell-Optik genannt), eine Halterung 7 für die IOL 8 und eine Sammellinse 9 lagert. Wird durch Drehung des Auges 4 eine unter einem Sichtwinkel $\alpha$ verkippte Sichtachse 5' eingenommen (Fig. 2), wird ein die Vorrichtung 1 passierender Lichtstrahl 10' - und damit das wahrnehmbare Bild - von der Iris 11 des Auges 4 zunehmend beschnitten (Fig. 3). Darüberhinaus weist das verbleibende, tatsächlich wahrgenommene Bild aufgrund von Defokussierung einen Kontrast und eine Auflösung auf, welche sich von dem Kontrast und der Auflösung der IOL 8 im tatsächlich implantierten Zustand - im Weiteren als "implantierte IOL" bezeichnet - deutlich unterscheiden, wie aus der in Fig. 4 dargestellten Modulationsübertragungsfunktion ("modulation transfer function", MTF) der Vorrichtung 1 für die Sichtwinkel $\alpha$ = 0° (strichpunktierte Linie 12), $\alpha$ = 2,5° (strichlierte Linie 13) und $\alpha$ = 5° (gepunktete Linie 14) einerseits und der Soll-MTF der implantierten IOL 8 (durchgezogene Linie 15) andererseits ersichtlich ist.

Aus all diesen Gründen ist es für Patienten noch immer schwierig, aus den vielfältigen Ausführungen von IOLs, seien es monofokale, bifokale, multifokale, EDOF- ("Extended Depth of Focus") -Linsen, lichtadjustierbare Linsen (LAL) etc., jene IOL auszuwählen, welche einen optimalen subjektiven Seheindruck gewährleistet.

Die Erfindung setzt sich zum Ziel, eine Vorrichtung zum Prüfen des Sehens durch eine IOL zu schaffen, welche die Nachteile des Standes der Technik überwindet und eine Linsenüberprüfung mit erweitertem Sichtfeld ermöglicht.

Dieses Ziel wird mit einer Vorrichtung der eingangs genannten Art erreicht, die sich erfindungsgemäß dadurch auszeichnet, dass die Okular-Optik distal eine Korrekturlinse und, proximal dazu, eine Zerstreuungslinse und eine Sammellinse umfasst, wobei die Korrekturlinse eine distale konkave Linsenoberfläche und eine proximale konvexe Linsenoberfläche aufweist.

Die erfindungsgemäße Kombination aus Korrekturlinse, Zerstreuungslinse und Sammellinse in der Okular-Optik erlaubt durch die spezielle Form der Linsenoberflächen, das Sichtfeld der Vorrichtung bzw. eines durch diese blickenden Auges eines Patienten zu erweitern und gleichzeitig Defokussierungen und Kontrastfehler im erweiterten Sichtfeld zu korrigieren und bestenfalls zu eliminieren.

So werden durch das erfindungsgemäße Zusammenspiel der konkav-konvexen Korrekturlinse und der nachgeschalteten Zerstreuungs- und Sammellinsen für unterschiedliche Einfallswinkel von Lichtstrahlen verschiedene Vergenzen und verschiedene Winkelunterschiede zwischen jeweiligem Eintritts- und Austrittsstrahl in Bezug auf die zentrale Sichtachse bzw. optische Achse erzeugt. Eben diese speziellen Vergenzen und Winkelunterschiede erlauben - in Kombination mit der Cornea-Optik - für unterschiedliche Sichtwinkel aus jeweiligen Raumwinkeln einfallende Lichtstrahlen im Wesentlichen auf denselben Punkt des Auges zu lenken und damit eine Sichtwinkel-Korrektur, d.h. eine fokussierte, mit der implantierten IOL in Auflösung und Kontrast übereinstimmende Sicht durch die IOL für unterschiedliche Sichtwinkel, zu erhalten.

Die Cornea-Optik kann dabei - in einer besonders effizienten Variante - eine durchschnittliche Hornhaut modellieren, **z.B.** gemäß dem Liou-Brennan-Augenmodell, um eine rasche Sichtprüfung durchzuführen; oder - in einer besonders genauen Variante - die Hornhaut des individuellen Patienten modellieren, **z.B.** nach deren vorheriger Vermessung, um dem individuellen Patienten eine möglichst realistische Prüfung der IOL zu ermöglichen.

Nicht zuletzt ermöglicht die erfindungsgemäße Vorrichtung das Überprüfen des subjektiven Seheindrucks durch die IOL in unterschiedlichen Situationen, sei es das Überprüfen der Fernsicht oder der Nahsicht. Dadurch eignet sich die erfindungsgemäße Vorrichtung insbesondere zur Überprüfung einer Vielfalt von Intraokularlinsen, z.B. monofokaler, bifokaler, multifokaler oder EDOF-Linsen. Um eine rasche Überprüfung einer Vielzahl unterschiedlicher Intraokularlinsen durchzuführen, kann die Halterung als austauschbare Küvette ausgeführt sein, in welcher die IOL angeordnet ist.

Zusammenfassend kann mithilfe der Vorrichtung der Erfindung einem Patienten schon vor der Implantation einer IOL ein realistischer subjektiver Seheindruck - als wäre die IOL implantiert - über ein größeres Sichtfeld gewährt und die Auswahl einer geeigneten IOL erleichtert werden.

In einer bevorzugten Ausführungsform ist zumindest eine der einander zugewandten Linsenoberflächen der Zerstreuungs- und Sammellinsen eine Asphäre. Im Zusammenspiel mit der konkav-konvexen Korrekturlinse und der Cornea-Optik kann dadurch eine besonders gute Sichtwinkelkorrektur, d.h. eine besonders gut fokussierte, mit der implantierten IOL für unterschiedliche Sichtwinkel in Auflösung und Kontrast übereinstimmende Sicht durch die IOL, erzielt werden.

In einer vorteilhaften Ausführungsform ist zumindest eine der einander zugewandten Linsenoberflächen der Zerstreuungs- und Sammellinsen eine Asphäre zumindest vierter Ordnung. Die Verwendung einer solchen asphärischen Linse erlaubt, Abbildungsfehler, wie sphärische Aberrationen, Astigmatismus, etc., zu vermeiden bzw. zu korrigieren. Dadurch kann dem Patienten über ein weites Sichtfeld ein fehlerfreier Blick durch die IOL und damit deren genaue Überprüfung ermöglicht werden. Während die Verwendung einer Asphäre höherer Ordnung eine große Flexibilität im Design der Okular-Optik bietet, ist eine Asphäre niedriger Ordnung einfacher herzustellen und deren Einfluss auf den Strahlengang einfacher vorherzusagen.

In einer besonders vorteilhaften Variante ist die Asphäre vierter Ordnung und die von der Scheitelebene der Asphäre weg gemessene Pfeilhöhe gewählt gemäß

$$z_1 = \frac{\rho \cdot r^2}{1 + \sqrt{1 - (\rho \cdot r)^2}} + A_2 \cdot r^2 + A_4 \cdot r^4 \quad \text{mit} \quad A_4 < 0, \qquad (1)$$

mit

$z_1$    Pfeilhöhe, von distal zu proximal gemessen,
$\rho$    Scheitelkrümmung,
r    Radialabstand von der zentralen Sichtachse, und
$A_2$, $A_4$    vorgegebene Koeffizienten, wobei $A_2 > 0$, wenn die Asphäre eine Linsenoberfläche der Sammellinse ist, und $A_2 < 0$, wenn die Asphäre eine Linsenoberfläche der Zerstreuungslinse ist.

Eine derartige Linsenoberfläche, deren Pfeilhöhe einer stetigen Funktion niedriger Ordnung folgt, kann einfach und präzise hergestellt werden.

Wenn die Asphäre die distale Linsenoberfläche der Sammellinse ist, können die Fertigung erleichtert und die erzielbare Sichtwinkel-Korrektur der Vorrichtung weiter gesteigert werden.

Um die Fertigung der Asphäre besonders zu vereinfachen, ist es günstig, wenn die der Asphäre gegenüberliegende Linsenoberfläche der Sammel- bzw. Zerstreuungslinse planar ist. Zu diesem Zweck kann die Sammellinse oder die Zerstreuungslinse optional aus mehreren, voneinander beabstandeten Einzellinsen zusammengesetzt sein.

In einer günstigen Variante weist zumindest eine der Cornea- und Okular-Optiken einen Achromat oder Apochromat auf, um chromatische Aberrationen zu vermeiden. Dadurch können Linsenüberprüfungen, welche mit der implantierten IOL in Auflösung, Fokussierung und Kontrast über ein großes Sichtfeld übereinstimmen, für eine Vielzahl an Wellenlängen durchgeführt werden. Ferner können mit einem Achromat bzw. Apochromat sphärische Aberrationen vermieden bzw. korrigiert werden. Beispielsweise kann in dieser Variante eine dispersionsfreie und vom Einfallswinkel unabhängige Kollimation eines die Vorrichtung durchlaufenden Lichtstrahls vor dem Auge des Patienten erzielt werden.

Die Cornea-Optik kann auf unterschiedliche Arten ausgeführt sein. In einer fertigungstechnisch besonders einfachen Variante weist die Cornea-Optik eine Cornea-Zerstreuungslinse und eine Cornea-Sammellinse auf, welche in beliebiger Reihenfolge entlang der optischen Sichtachse angeordnet sein können.

Wenn die Sammel- und Zerstreuungslinsen der Cornea-Optik und/oder jene der Okular-Optik unterschiedliche Abbe-Zahlen haben, bilden die jeweiligen Sammel- und Zerstreuungslinsen einen Achromat bzw. Apochromat und können so neben den sphärischen auch chromatische Aberrationen korrigieren bzw. eliminieren. Damit wird dem Patienten ermöglicht, die IOL einer besonders genauen Überprüfung zu unterziehen und bereits vor deren Implantation über die Qualität der IOL sowohl hinsichtlich der Schärfe als auch hinsichtlich der Farbwahrnehmung zu urteilen und so eine geeignete IOL zu finden.

In Versuchen hat sich gezeigt, dass es günstig ist, wenn die distale Linsenoberfläche der Cornea-Sammellinse eine Asphäre zumindest vierter Ordnung ist. Im Zusammenspiel mit der zumindest einen Asphäre und der Korrekturlinse der Okular-Optik kann so **z.B.** eine Sichtwinkel-korrigierte Linsenüberprüfung für Sichtwinkel von über 10° erhalten werden, wodurch dem Patienten eine besonders realistische Linsenüberprüfung über ein weites Sichtfeld ermöglicht wird.

Dabei ist es besonders günstig, wenn die von der Scheitelebene der distalen Linsenoberfläche der Cornea-Sammellinse weg gemessene Pfeilhöhe dieser distalen Linsenoberfläche gewählt ist gemäß

$$z_2 = \frac{\rho \cdot r^2}{1 + \sqrt{1 - (\rho \cdot r)^2}} + A_2 \cdot r^2 + A_4 \cdot r^4 \quad \text{mit} \quad A_2 > 0 \quad \text{und} \quad A_4 < 0, \qquad (2)$$

mit

$z_2$      Pfeilhöhe, von distal zu proximal gemessen,
$\rho$      Scheitelkrümmung,
$r$      Radialabstand von der zentralen Sichtachse, und
$A_2, A_4$      vorgegebene Koeffizienten.

Eine derartige distale Linsenoberfläche, deren Pfeilhöhe einer stetigen Funktion niedriger Ordnung folgt, kann einfach und präzise hergestellt werden.

Auch die Korrekturlinse der Okular-Optik kann auf unterschiedliche Arten ausgeführt sein. In einer bevorzugten Ausführungsform sind die distale und die proximale Linsenoberfläche der Korrekturlinse Sphären und der Krümmungsradius der distalen Linsenoberfläche der Korrekturlinse ist betragsmäßig kleiner als der Krümmungsradius der proximalen Linsenoberfläche der Korrekturlinse. Mit dieser Korrekturlinse kann - im Zusammenspiel mit der vorgeschalteten Cornea-Optik und den nachgeschalteten Zerstreuungs- und Sammellinsen der Okular-Optik - ein stark erweitertes Sichtfeld unter Vermeidung von Defokussierungen und Kontrastfehlern im erweiterten Sichtfeld erzielt werden.

In allen Ausführungsformen ist es vorteilhaft, wenn zumindest eines der Elemente Cornea-Optik, Halterung und Okular-Optik entlang der zentralen Sichtachse auf dem Träger verstellbar gelagert ist. Dabei kann jedes der genannten Elemente entweder als Ganzes verstellt werden oder, wenn eines der Elemente Sub-Elemente aufweist, z.B. die genannten Korrektur-, Sammel- und Zerstreuungslinsen der Okular-Optik, auch die Sub-Elemente gesondert verstellt werden. Eine verstellbare Lagerung ermöglicht eine schnelle Anpassung der Vorrichtung, beispielsweise eine Verschiebung der optischen Hauptebenen einer Optik, an das gerade sichtprüfende Auge des Patienten. Dadurch kann die Vergenz des aus der Okular-Optik proximal austretenden Lichtstrahls an die Fehlsichtigkeit des Patienten angepasst werden, beispielsweise zur Korrektur sphärischer Refraktionsfehler. Durch diese Anpassung kann eine Linsenüberprüfung, welche hinsichtlich Kontrast, Auflösung und Fokussierung die IOL im implantierten Zustand nachbildet, auch für fehlsichtige Patienten gewährleistet werden.

Ebenso ist es vorteilhaft, wenn zumindest eines der Elemente Cornea-Optik, Halterung und Okular-Optik quer zur zentralen Sichtachse auf dem Träger verstellbar gelagert ist, um die Vorrichtung einfach justieren und kalibrieren zu können.

Nicht zuletzt ist es günstig, wenn die Halterung dazu ausgebildet ist, die Intraokularlinse austauschbar aufzunehmen. Eine derartige Schnellwechselhalterung erlaubt es, eine bereits überprüfte IOL in der Vorrichtung in kurzer Zeit durch eine neue zu überprüfende IOL auszutauschen. Durch den raschen Austausch wird es dem Patienten ermöglicht, eine Vielzahl unterschiedlicher IOLs effizient zu überprüfen und so die für ihn am besten geeignete IOL anhand des subjektiven Seheindrucks für eine anschließende Implantation auszuwählen.

In einem weiteren Aspekt der Erfindung wird die Verwendung einer Okular-Optik, welche distal eine Korrekturlinse und, proximal dazu, eine Zerstreuungslinse und eine Sammellinse umfasst, wobei die Korrekturlinse eine distale konkave Linsenoberfläche und eine proximale konvexe Linsenoberfläche aufweist, und wobei bevorzugt zumindest eine der einander zugewandten Linsenoberflächen der Zerstreuungs- und Sammellinsen eine Asphäre ist, in einer Vorrichtung zum Prüfen des Sehens durch eine Intraokularlinse bereitgestellt. Bezüglich der Vorteile und möglicher Ausgestaltungen der Verwendung der Okular-Optik in einer optischen Prüfvorrichtung wird auf die obigen Ausführungen zur erfindungsgemäßen Vorrichtung verwiesen.

Die Erfindung wird nachfolgend anhand von in den beigeschlossenen Zeichnungen dargestellten Ausführungsbeispielen näher erläutert. In den Zeichnungen zeigen:

Fig. 1 eine Vorrichtung zum Prüfen des Sehens durch eine Intraokularlinse gemäß dem Stand der Technik mit beispielhaftem Strahlengang für ein in zentraler Sichtachse auf die Intraokularlinse blickendes Auge in einer schematischen Seitenansicht;

Fig. 2 die Vorrichtung von Fig. 1 mit beispielhaftem Strahlengang für ein unter einem Sichtwinkel auf die Intraokularlinse blickendes Auge in einer schematischen Seitenansicht;

Fig. 3 das Auge von Fig. 2 und einen defokussierten und von der Iris des Auges beschnittenen Lichtstrahl im Strahlengang von Fig. 2 in einer schematischen Seitenansicht;

Fig. 4 beispielhafte Modulationsübertragungsfunktionen, wie sie für unterschiedliche Sichtwinkel mit der Vorrichtung der Fig. 1 und 2 erzielt werden, in einem Kontrast-Linien-Diagramm;

Fig. 5 eine Vorrichtung zum Prüfen des Sehens durch eine Intraokularlinse gemäß der vorliegenden Erfindung mit beispielhaftem Strahlengang für ein in zentraler Sichtachse auf die Intraokularlinse blickendes Auge in einer schematischen Seitenansicht;

Fig. 6 die Vorrichtung von Fig. 5 mit beispielhaftem Strahlengang für ein unter einem Sichtwinkel auf die Intraokularlinse blickendes Auge in einer schematischen Seitenansicht;

Fig. 7 das Auge von Fig. 6 und einen fokussierten und von der Iris des Auges nicht beschnittenen Lichtstrahl im Strahlengang von Fig. 6 in einer schematischen Seitenansicht;

Fig. 8 beispielhafte Modulationsübertragungsfunktionen, wie sie für unterschiedliche Sichtwinkel mit der Vorrichtung der Fig. 5 und 6 erzielt werden, in einem Kontrast-Linien-Diagramm;

Fig. 9 die Okular-Optik der Vorrichtung der Fig. 5 und 6 in einer seitlichen Explosionsdarstellung;

Fig. 10 die Cornea-Optik der Vorrichtung der Fig. 5 und 6 in einer seitlichen Explosionsdarstellung;

Fig. 11 beispielhafte chromatische Aberrationen, wie sie bei der Vorrichtung der Fig. 1 bis 3 bzw. bei der Vorrichtung der Fig. 5 bis 7 auftreten, in einem Diagramm der longitudinalen chromatischen Aberration über der Wellenlänge; und

Fig. 12 beispielhafte sphärische Aberrationen, wie sie bei der Vorrichtung der Fig. 1 bis 3 bzw. bei der Vorrichtung der Fig. 5 bis 7 auftreten, in einem Diagramm des Zernike-Koeffizienten über dem Sichtwinkel.

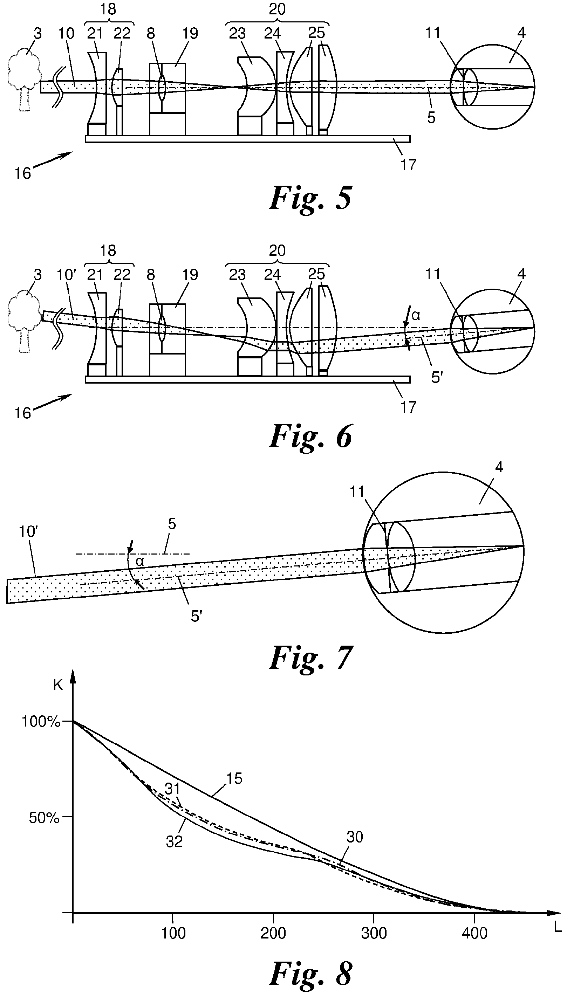

Anhand der Fig. 1 bis 4 wurde bereits weiter oben eine herkömmliche optische Prüfvorrichtung 1 für eine Intraokularlinse (IOL) 8, wie sie im Stand der Technik bekannt ist, beschrieben. In den Fig. 5 bis 12 bezeichnen gleiche Bezugszeichen gleiche Teile wie in den Fig. 1 bis 4.

Die Fig. 5 bis 7 zeigen eine erfindungsgemäße optische Prüfvorrichtung 16 zum Prüfen des Sehens durch die IOL 8 vor deren Implantation in das Auge 4 eines Patienten. Die zu prüfende IOL 8, welche nach ihrer Implantation - im Folgenden als "implantierte IOL" bezeichnet - zur Sichtverbesserung des Patienten führen soll, kann z.B. eine monofokale, bifokale, multifokale, EDOF- ("Extended Depth of Focus") -Linse, eine lichtadjustierbare Linse (LAL), etc. sein.

Um dem Patienten einen subjektiven Seheindruck durch die noch nicht implantierte IOL 8 auf eine nahe, intermediäre oder ferne Umgebung 3 zu verschaffen, umfasst die Vorrichtung 16 einen Träger 17, der von distal (nahe zur betrachteten Umgebung 3) bis proximal (nahe zum Auge 4 eines Patienten) entlang einer zentralen Sichtachse 5 aufeinanderfolgend eine Cornea-Optik 18, eine Halterung 19 für die IOL 8 und eine Okular-Optik 20 trägt. Der Träger 17 kann dafür jede in der Optik bekannte Vorrichtung zum Lagern optischer Elemente sein, z.B. ein Rahmen, ein Gehäuse, ein Rohr, eine optische Bank, ein optischer Tisch, usw. Der Träger 17 kann die optischen Elemente entweder in fester Stellung oder verstellbar lagern. Beispielsweise können die Cornea-Optik 18 (oder Teile davon), die Halterung 19 (oder Teile davon) und/oder die Okular-Optik 20 (oder Teile davon) entlang der zentralen Sichtachse 5 verschieblich und/oder normal dazu verstellbar am Träger 17 gelagert sein, um sie relativ zueinander zu justieren.

Die optische Wirkung der Vorrichtung 16 soll im Folgenden bei gerader Durchsicht entlang der zentralen Sichtachse 5 (Fig. 5) und bei schräger Durchsicht entlang einer unter einem Sichtwinkel $\alpha$ geneigten Sichtachse 5' (Fig. 6) anhand zweier repräsentativer Lichtstrahlen 10 (Fig. 5) bzw. 10' (Fig. 6) beschrieben werden.

Zuerst passiert ein von der Umgebung 3, d.h. vom distalen Ende der Vorrichtung 16 her, einfallender Lichtstrahl 10 bzw. 10' die Cornea-Optik 18, welche eine Hornhaut optisch nachbildet, im gezeigten Beispiel durch eine distale Cornea-Zerstreuungslinse 21 und eine proximale Cornea-Sammellinse 22. In einer ersten Variante bildet die Cornea-Optik 18 eine Hornhaut eines "Durchschnittspatienten", **z.B.** gemäß einem Augenmodell wie beispielsweise dem Liou-Brennan-Augenmodell. In einer alternativen Variante bildet die Cornea-Optik 18 die Hornhaut des Auges 4 eines individuellen Patienten nach, wozu diese vorher vermessen wird, z.B. optisch oder mittels Ultraschalls.

Nach der Cornea-Optik 18 wird der Lichtstrahl 10 bzw. 10' durch die IOL 8 in der Halterung 19 geführt, wo sie - so als ob sie im Auge 4 implantiert wäre - den Lichtstrahl 10 bzw. 10' ihrer Brechkraft gemäß fokussiert.

Die Halterung 19 kann jede zur Aufnahme der IOL 8 geeignete Halterung sein, z.B. ein modulares Schnellwechselsystem mit einem am Träger 17 fest gelagerten ersten Teil zur Aufnahme eines schnell austauschbaren zweiten Teils, in oder an welchem die IOL 8 aufgenommen ist, um verschiedenste IOLs durch Wechseln des zweiten Teils rasch überprüfen zu können. Beispielsweise kann die Halterung 19 als ersten Teil eine Klemm-, Steck- oder Magnethalterung aufweisen, welche eine wechselbare Küvette für die IOL, optional gefüllt mit einer Lösung zur Nachbildung des Augeninneren, als zweiten Teil aufnimmt.

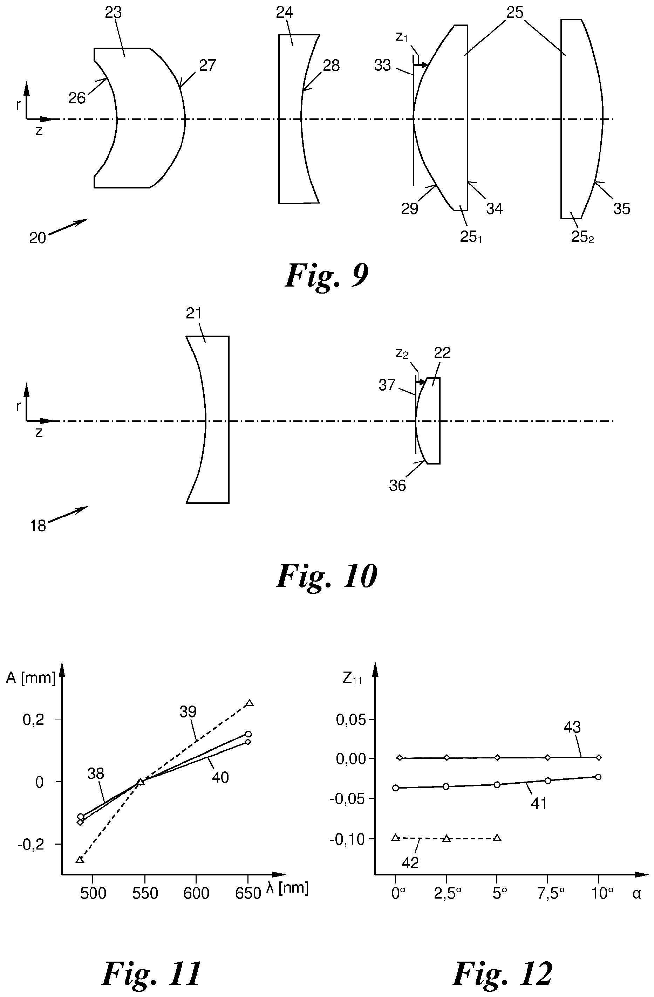

Anschließend durchquert der Lichtstrahl 10 bzw. 10' die Okular-Optik 20, welche eine distale Korrekturlinse 23 und, proximal dazu, eine Zerstreuungslinse 24 und eine Sammellinse 25 umfasst. Alternativ zur gezeigten Ausführungsform kann die Sammellinse 25 distal zur Zerstreuungslinse 24 angeordnet sein. Die Korrekturlinse 23 hat eine distale konkave Linsenoberfläche 26 und eine proximale konvexe Linsenoberfläche 27, siehe Fig. 9. Optional ist zumindest eine oder beide der einander zugewandten Linsenoberflächen 28, 29 der Zerstreuungs- und Sammellinsen 24, 25 eine

EP 4 331 469 B1

Asphäre (hier: die distale Linsenoberfläche 29 der Sammellinse 25).

Durch diese speziellen Korrektur-, Zerstreuungs- und Sammellinsen 23 - 25 werden die Lichtstrahlen 10 bzw. 10' in der Okular-Optik 20 abhängig vom Sichtwinkel $\alpha$ in Vergenz und Richtung derart verändert, dass sie nach Durchqueren der Okular-Optik 20 sowohl bei gerader (Fig. 5: $\alpha = 0°$) als auch bei geneigter Sichtachse (Fig. 6 und 7: $\alpha \neq 0°$) von der Iris 11 des Auges 4 nicht beschnitten werden. Selbstverständlich werden bei fixem Sichtwinkel $\alpha$ auch unter leicht verschiedenen Winkeln einfallende Lichtstrahlen von der Iris 11 nicht beschnitten, woraufhin sie auch auf periphere Punkte der Netzhaut des Auges 4 fokussiert werden.

Wie aus einem Vergleich der Fig. 3 und 7 ersichtlich, wird durch das Zusammenspiel der Korrektur-, Zerstreuungs- und Sammellinsen 23 - 25 der Okular-Optik 20 der entlang der geneigten Sichtachse 5' propagierende Lichtstrahl 10' in der Vorrichtung 16 nicht mehr von der Iris 11 des Auges 4 beschnitten und korrekt auf der Netzhaut des Auges 4 fokussiert. In der Folge ist das unter einem Sichtwinkel $\alpha > 0°$ wahrgenommene Bild nicht nur unbeschnitten, sondern mit der implantierten IOL 8 in Kontrast und Auflösung besser übereinstimmend als jenes der herkömmlichen Vorrichtung 1, wie aus einem Vergleich der in Fig. 4 gezeigten Modulationsübertragungsfunktionen der Vorrichtung 1 mit den in Fig. 8 gezeigten Modulationsübertragungsfunktionen der Vorrichtung 16 für $\alpha = 0°$ (strichpunktierte Linie 30), $\alpha = 2,5°$ (strichlierte Linie 31) und $\alpha = 5°$ (gepunktete Linie 32), jeweils mit der Soll-MTF der implantierten IOL 8 (durchgezogene Linie 15), in Diagrammen des Kontrastes K über Linienpaaren L hervorgeht. Somit kann in der erfindungsgemäßen Vorrichtung 16 ein größeres Sichtfeld der IOL 8 als mit der herkömmlichen Vorrichtung 1 überprüft werden.

In der in den Fig. 5, 6 und 9 gezeigten Ausführungsform der Okular-Optik 20 ist die zumindest eine Asphäre der Zerstreuungs- und Sammellinsen 24, 25 durch die distale Linsenoberfläche 29 der Sammellinse 25 gebildet. Asphären können nach DIN ISO 10110 allgemein durch die von der Scheitelebene der Linsenoberfläche weg gemessene Pfeilhöhe z in Abhängigkeit vom Radialabstand r zur zentralen Sichtachse 5 beschrieben werden gemäß

$$z = \frac{\rho \cdot r^2}{1 + \sqrt{1 - (1+k) \cdot (\rho \cdot r)^2}} + \sum_{i=1}^{I} A_i \cdot r^i \qquad (3)$$

mit

$\rho$     Scheitelkrümmung,
k     konische Konstante, und
$A_i$     vorgegebene Koeffizienten.

In einer Variante der asphärischen distalen Linsenoberfläche 29 ist diese eine Asphäre zumindest vierter Ordnung, d.h. zumindest ein Koeffizient $A_i$ mit $i \geq 4$ in Gleichung (3) ist ungleich Null, wobei der erste Summand entweder vorhanden sein kann oder nicht. In einer beispielhaften Untervariante davon ist die von der Scheitelebene 33 der distalen Linsenoberfläche 29 weg gemessene Pfeilhöhe $z_1$ der distalen Linsenoberfläche 29 gewählt gemäß

$$z_1 = \frac{\rho \cdot r^2}{1 + \sqrt{1 - (\rho \cdot r)^2}} + A_2 \cdot r^2 + A_4 \cdot r^4 \quad \text{mit} \quad A_2 > 0 \quad \text{und} \quad A_4 < 0 , \quad (1)$$

mit

$z_1$     Pfeilhöhe der distalen Linsenoberfläche 29, von distal zu proximal gemessen,
$\rho$     Scheitelkrümmung,
r     Radialabstand von der zentralen Sichtachse 5, und
$A_2, A_4$     vorgegebene Koeffizienten.

In einer alternativen Variante der Okular-Optik 20, in welcher deren Asphäre durch die proximale Linsenoberfläche 28 der Zerstreuungslinse 24 gebildet ist, ist diese Linsenoberfläche 28 eine Asphäre zumindest vierter Ordnung, und in einer beispielhaften Untervariante davon ist die von der Scheitelebene der proximalen Linsenoberfläche 28 weg gemessene Pfeilhöhe der distalen Linsenoberfläche gewählt gemäß

$$z_{1'} = \frac{\rho \cdot r^2}{1 + \sqrt{1 - (\rho \cdot r)^2}} + A_2 \cdot r^2 + A_4 \cdot r^4 \quad \text{mit} \quad A_2 < 0 \quad \text{und} \quad A_4 < 0, \quad (1')$$

mit

z₁'  Pfeilhöhe der proximalen Linsenoberfläche 28, von distal zu proximal gemessen,

ρ  Scheitelkrümmung,

r  Radialabstand von der zentralen Sichtachse 5, und

A₂, A₄  vorgegebene Koeffizienten.

In der gezeigten Ausführungsform ist optional die Zerstreuungslinse 24 planar-konkav und die Sammellinse 25 aus einem ersten konvex-planaren Segment 25₁ und einem zweiten planar-konvexen Segment 25₂ gebildet. Dadurch ist die der asphärischen Linsenoberfläche 29 gegenüberliegende Linsenoberfläche 34 planar und die Asphäre einfacher herzustellen. Ferner ist durch die Krümmung der proximalsten Linsenoberfläche (hier: der proximalen Linsenoberfläche 35 des proximalen Segments 25₂) der Lichtstrahl 10 bzw. 10' kollimiert auf das Auge 4 gerichtet.

In weiteren optionalen Ausführungsformen können die Sammel- und Zerstreuungslinsen 24, 25 unterschiedliche Abbe-Zahlen haben, um einen Achromat zu bilden.

Zur Sichtwinkel-Vergrößerung kann die konkav-konvexe Korrekturlinse 23 auf viele Weisen ausgestaltet sein, z.B. können deren distale und proximale Linsenoberflächen 26, 27 jeweils für sich sphärisch oder asphärisch sein. In der in Fig. 9 gezeigten Variante sind die beiden Linsenoberflächen 26, 27 der Korrekturlinse 23 Sphären. Darüberhinaus ist die distale Linsenoberfläche 26 stärker gekrümmt als die proximale Linsenoberfläche 27, also der Krümmungsradius der Ersteren betragsmäßig kleiner als der Krümmungsradius der Letzteren.

Auch die Cornea-Optik 18 kann auf viele Weisen ausgestaltet sein. In der in den Fig. 5, 6 und 10 gezeigten Ausführungsform ist die Cornea-Optik 18 durch eine (hier: konkav-planare) Cornea-Zerstreuungslinse 21 und eine (hier: konvex-planare) Cornea-Sammellinse 22 gebildet, welche entweder in der dargestellten Reihenfolge mit distaler Cornea-Zerstreuungs- und proximaler Cornea-Sammellinse 21, 22 oder in umgekehrter Reichenfolge mit distaler Cornea-Sammel- und proximaler Cornea-Zerstreuungslinse 21, 22 angeordnet sein können.

In einer Variante davon weisen die Cornea-Zerstreuungslinse 21 und die Cornea-Sammellinse 22 unterschiedliche Abbe-Zahlen auf und bilden so einen Achromat, der chromatische Aberrationen, optional zusätzlich zu sphärischen Aberrationen, korrigiert.

Ferner ist in der gezeigten Ausführungsform die distale Linsenoberfläche 36 der Cornea-Sammellinse 22 eine Asphäre. In einer Variante ist diese Asphäre vierter Ordnung, und in einer beispielhaften Untervariante davon ist die von der Scheitelebene 37 der distalen Linsenoberfläche 36 weg gemessene Pfeilhöhe z₂ der distalen Linsenoberfläche 36 gewählt gemäß

$$z_2 = \frac{\rho \cdot r^2}{1+\sqrt{1-(\rho \cdot r)^2}} + A_2 \cdot r^2 + A_4 \cdot r^4 \quad \text{mit} \quad A_2 > 0 \quad \text{und} \quad A_4 < 0, \qquad (2)$$

mit

z₂  Pfeilhöhe, von distal zu proximal gemessen,

ρ  Scheitelkrümmung,

r  Radialabstand von der zentralen Sichtachse 5, und

A₂, A₄  vorgegebene Koeffizienten.

Optional oder alternativ dazu könnte die Cornea-Zerstreuungslinse 21 eine asphärische Linsenoberfläche aufweisen.

Selbstverständlich sind auch andere Ausgestaltungen der Cornea-Optik 18 und/oder der Okular-Optik 20 mit weiteren oder anderen optischen Elementen möglich, z.B. mit einem Achromat oder Apochromat zur Farbkorrektur und/oder einer proximalen Linse zur Fokussierung oder Defokussierung, mit Spiegeln zur Strahlablenkung, usw.

Ferner ist festzuhalten, dass jede der Linsen 21 - 25 sowohl einstückig als auch durch mehrere - optional voneinander getrennte - Segmente ausgeführt sein kann, wie hier bei der Linse 25 gezeigt.

In Versuchen konnten mit einer asphärischen Linsenoberfläche 36 der Cornea-Sammellinse 22 mit einer Scheitelkrümmung ρ zwischen 0,058/mm und 0,118/mm, bevorzugt 0,088/mm, einem Koeffizienten $A_2$ zwischen $1 \cdot 10^{-5}$/mm und $4{,}4 \cdot 10^{-5}$/mm, bevorzugt $2{,}7 \cdot 10^{-5}$/mm, einem Koeffizienten $A_4$ zwischen $-0{,}6 \cdot 10^{-4}$/mm³ und $-1{,}6 \cdot 10^{-4}$/mm³, bevorzugt $-1{,}1 \cdot 10^{-4}$/mm³; und mit einer asphärischen Linsenoberfläche 29 der Sammellinse 25 mit einer Scheitelkrümmung ρ zwischen 0,01/mm und 0,09/mm, bevorzugt 0, 05/mm, einem Koeffizienten $A_2$ zwischen 0,005/mm und 0,025/mm, bevorzugt 0, 015/mm, einem Koeffizienten $A_4$ zwischen $-1 \cdot 10^{-5}$/mm³ und $-9 \cdot 10^{-5}$/mm³, bevorzugt $-5 \cdot 10^{-5}$/mm³; und mit einer Korrekturlinse 23 mit sphärischer distaler Linsenoberfläche 26 mit einem Krümmungsradius zwischen -18 mm und -7 mm, bevorzugt -12,5 mm, und mit sphärischer proximaler Linsenoberfläche 27 mit einem Krümmungsradius zwischen -19 mm und -7 mm, bevorzugt -13 mm, besonders gute Sichtfelderweiterungen erzielt werden.

Die Fig. 11 und 12 veranschaulichen die in Versuchen mit der Vorrichtung 16 gegenüber der herkömmlichen Vorrichtung 1 erzielte Verbesserung der longitudinalen chromatischen Aberration (hier: der Fokusvariation A über der Wellenlänge $\lambda$) entlang der zentralen Sichtachse 5 (Fig. 11) und der sphärischen Aberration anhand des Zernike-Koeffizienten $Z_{11}$ bei einer Wellenlänge von 546 nm für unterschiedliche Sichtwinkel $\alpha$ (Fig. 12). Dabei entsprechen die durchgezogenen Linien mit Kreisen 38 bzw. 41 den Ergebnissen der erfindungsgemäßen Vorrichtung 16, die strichlierten Linien mit Dreiecken 39 bzw. 42 der herkömmlichen Vorrichtung 1 und die gepunkteten Linien mit Rauten 40 bzw. 43 den Ergebnissen der implantierten IOL 8.

Falls gewünscht, können entlang der zentralen Sichtachse 5 noch zusätzliche optische Elemente angeordnet sein, z.B. um Aberrationen zu korrigieren oder die möglichen Sichtwinkel $\alpha$ zur Prüfung der IOL 8 zu erweitern. Ferner kann die Vorrichtung 16 alternativ auch ganz oder teilweise mit nichtrotationssymmetrischen Linsen aufgebaut sein, beispielsweise die hierin beschriebenen asphärischen Linsenoberflächen der Cornea-Optik 18 und/oder der Okular-Optik 20 durch Systeme aus mehreren sphärischen Linsenoberflächen ersetzt sein, welche eine vergleichbare optische Wirkung erzielen.

Nicht zuletzt können selbstverständlich mit einem Aufbau aus zwei parallelen Vorrichtungen 16 zwei IOLs 8 gleichzeitig und in binokularer (stereoskopischer) Sicht überprüft werden.

Ganz allgemein kann die Okular-Optik 20, optional kombiniert mit der Cornea-Optik 18, in einer beliebigen Vorrichtung zum Prüfen des Sehens durch eine IOL 8 verwendet werden.

Die Erfindung ist nicht auf die dargestellten Ausführungsformen beschränkt, sondern umfasst alle Varianten, Modifikationen und Kombinationen, die in den Rahmen der angeschlossenen Ansprüche fallen.

**Patentansprüche**

1. Vorrichtung zum Prüfen des Sehens durch eine Intraokularlinse (8), mit einem Träger (17), der in einer zentralen Sichtachse (5) von distal zu proximal aufeinanderfolgend eine Cornea-Optik (18), eine Halterung (19) für die Intraokularlinse (8) und eine Okular-Optik (20) lagert,

   **dadurch gekennzeichnet, dass** die Okular-Optik (20) distal eine Korrekturlinse (23) und, proximal dazu, eine Zerstreuungslinse (24) und eine Sammellinse (25) umfasst,
   wobei die Korrekturlinse (23) eine distale konkave Linsenoberfläche (26) und eine proximale konvexe Linsenoberfläche (27) aufweist.

2. Vorrichtung nach Anspruch 1, **dadurch gekennzeichnet, dass** zumindest eine der einander zugewandten Linsenoberflächen (28, 29) der Zerstreuungs- und Sammellinsen (24, 25) eine Asphäre ist.

3. Vorrichtung nach Anspruch 2, **dadurch gekennzeichnet, dass** zumindest eine der einander zugewandten Linsenoberflächen (28, 29) der Zerstreuungs- und Sammellinsen (24, 25) eine Asphäre zumindest vierter Ordnung ist.

4. Vorrichtung nach Anspruch 3, **dadurch gekennzeichnet, dass** die von der Scheitelebene (33) der Asphäre weg gemessene Pfeilhöhe ($z_1$) gewählt ist gemäß

$$z_1 = \frac{\rho \cdot r^2}{1 + \sqrt{1 - (\rho \cdot r)^2}} + A_2 \cdot r^2 + A_4 \cdot r^4 \quad \texttt{mit} \quad A_4 < 0 \texttt{,}$$

   mit

   $z_1$ Pfeilhöhe, von distal zu proximal gemessen,
   $\rho$ Scheitelkrümmung,
   r Radialabstand von der zentralen Sichtachse (5), und
   $A_2$, $A_4$ vorgegebene Koeffizienten, wobei $A_2 > 0$, wenn die Asphäre eine Linsenoberfläche der Sammellinse (24) ist, und $A_2 < 0$, wenn die Asphäre eine Linsenoberfläche der Zerstreuungslinse (25) ist.

5. Vorrichtung nach einem der Ansprüche 2 bis 4, **dadurch gekennzeichnet, dass** die Asphäre die distale Linsenoberfläche (29) der Sammellinse (25) ist.

**6.** Vorrichtung nach einem der Ansprüche 2 bis 5, **dadurch gekennzeichnet, dass** die der Asphäre gegenüberliegende Linsenoberfläche (34) der Sammel- bzw. Zerstreuungslinse (25, 24) planar ist.

**7.** Vorrichtung nach einem der Ansprüche 1 bis 6, **dadurch gekennzeichnet, dass** zumindest eine der Cornea- und Okular-Optiken (18, 20) einen Achromat (21, 22; 24, 25) oder Apochromat aufweist.

**8.** Vorrichtung nach einem der Ansprüche 1 bis 7, **dadurch gekennzeichnet, dass** die Cornea-Optik (18) eine Cornea-Zerstreuungslinse (21) und eine Cornea-Sammellinse (22) aufweist.

**9.** Vorrichtung nach den Ansprüchen 7 und 8, **dadurch gekennzeichnet, dass** die Cornea-Zerstreuungslinse (21) und die Cornea-Sammellinse (22) unterschiedliche Abbe-Zahlen haben und/oder die Sammel- und Zerstreuungslinsen (24, 25) der Okular-Optik (20) unterschiedliche Abbe-Zahlen haben.

**10.** Vorrichtung nach Anspruch 9, **dadurch gekennzeichnet, dass** die distale Linsenoberfläche (36) der Cornea-Sammellinse (22) eine Asphäre zumindest vierter Ordnung ist.

**11.** Vorrichtung nach Anspruch 10, **dadurch gekennzeichnet, dass** die von der Scheitelebene (37) der distalen Linsenoberfläche (36) der Cornea-Sammellinse (22) weg gemessene Pfeilhöhe ($z_2$) dieser distalen Linsenoberfläche gewählt ist gemäß

$$z_2 = \frac{\rho \cdot r^2}{1 + \sqrt{1 - (\rho \cdot r)^2}} + A_2 \cdot r^2 + A_4 \cdot r^4 \quad \text{mit} \quad A_2 > 0 \quad \text{und} \quad A_4 < 0,$$

mit

$z_2$ Pfeilhöhe, von distal zu proximal gemessen,
$\rho$ Scheitelkrümmung,
$r$ Radialabstand von der zentralen Sichtachse (5), und
$A_2$, $A_4$ vorgegebene Koeffizienten.

**12.** Vorrichtung nach einem der Ansprüche 1 bis 11, **dadurch gekennzeichnet, dass** die distale und die proximale Linsenoberfläche (26, 27) der Korrekturlinse (23) Sphären sind, wobei der Krümmungsradius der distalen Linsenoberfläche (26) der Korrekturlinse (23) betragsmäßig kleiner als der Krümmungsradius der proximalen Linsenoberfläche (27) der Korrekturlinse (23) ist.

**13.** Vorrichtung nach einem der Ansprüche 1 bis 12, **dadurch gekennzeichnet, dass** zumindest eines der Elemente Cornea-Optik (18), Halterung (19) und Okular-Optik (20) entlang der zentralen Sichtachse (5) und/oder quer zur zentralen Sichtachse (5) auf dem Träger (17) verstellbar gelagert ist.

**14.** Vorrichtung nach einem der Ansprüche 1 bis 13, **dadurch gekennzeichnet, dass** die Halterung (19) dazu ausgebildet ist, die Intraokularlinse (8) austauschbar aufzunehmen.

**15.** Verwenden einer Okular-Optik (20), welche distal eine Korrekturlinse (23) und, proximal dazu, eine Zerstreuungslinse (24) und eine Sammellinse (25) umfasst, wobei die Korrekturlinse (23) eine distale konkave Linsenoberfläche (26) und eine proximale konvexe Linsenoberfläche (27) aufweist, und wobei bevorzugt zumindest eine der einander zugewandten Linsenoberflächen (28, 29) der Zerstreuungs- und Sammellinsen (24, 25) eine Asphäre ist, in einer Vorrichtung (16) zum Prüfen des Sehens durch eine Intraokularlinse (8).

## Claims

**1.** A device for testing vision through an intraocular lens (8), with a carrier (17) which supports a corneal optics (18), a holder (19) for the intraocular lens (8), and an ocular optics (20) in succession from distal to proximal in a central viewing axis (5),

**characterised in that** the ocular optics (20) comprises distally a corrective lens (23) and, proximal thereto, a diverging lens (24) and a converging lens (25),
wherein the corrective lens (23) has a distal concave lens surface (26) and a proximal convex lens surface (27).

2. The device according to claim 1, **characterised in that** at least one of the mutually facing lens surfaces (28, 29) of the diffusing and converging lenses (24, 25) is an asphere.

3. The device according to claim 2, **characterised in that** at least one of the mutually facing lens surfaces (28, 29) of the diffusing and converging lenses (24, 25) is an asphere of at least the fourth order.

4. The device according to claim 3, **characterised in that** the arrow height ($z_1$) measured away from the apex plane (33) of the asphere is selected according to

$$z_1 = \frac{\rho \cdot r^2}{1 + \sqrt{1 - (\rho \cdot r)^2}} + A_2 \cdot r^2 + A_4 \cdot r^4 \;\; \text{with} \;\; A_4 < 0,$$

with

$z_1$ arrow height, measured from distal to proximal,
$\rho$ apex curvature,
r radial distance from the central viewing axis (5), and
$A_2$, $A_4$ predetermined coefficients, wherein $A_2 > 0$, if the asphere is a lens surface of the converging lens (24), and $A_2 < 0$, if the asphere is a lens surface of the diverging lens (25).

5. The device according to any one of claims 2 to 4, **characterised in that** the asphere is the distal lens surface (29) of the converging lens (25).

6. The device according to any one of claims 2 to 5, **characterised in that** the lens surface (34) of the converging or diverging lens (25, 24) opposite the asphere is planar.

7. The device according to any one of claims 1 to 6, **characterised in that** at least one of the corneal and ocular optics (18, 20) comprises an achromat (21, 22; 24, 25) or apochromat.

8. The device according to any one of claims 1 to 7, **characterised in that** the corneal optics (18) comprises a corneal diverging lens (21) and a corneal converging lens (22).

9. The device according to claims 7 and 8, **characterised in that** the corneal diverging lens (21) and the corneal converging lens (22) have different Abbe numbers and/or the converging and diverging lenses (24, 25) of the ocular optics (20) have different Abbe numbers.

10. The device according to claim 9, **characterised in that** the distal lens surface (36) of the corneal converging lens (22) is an asphere of at least the fourth order.

11. The device according to claim 10, **characterised in that** the arrow height ($z_2$) of this distal lens surface measured away from the apex plane (37) of the distal lens surface (36) of the corneal converging lens (22) is selected according to

$$z_2 = \frac{\rho \cdot r^2}{1 + \sqrt{1 - (\rho \cdot r)^2}} + A_2 \cdot r^2 + A_4 \cdot r^4 \, \text{with} \;\; A_2 > 0 \;\; \text{and} \;\; A_4 < 0,$$

with

$z_2$ arrow height, measured from distal to proximal,
$\rho$ apex curvature,
r radial distance from the central viewing axis (5), and
$A_2$, $A_4$ predetermined coefficients.

**12.** The device according to any one of claims 1 to 11, **characterised in that** the distal and proximal lens surfaces (26, 27) of the corrective lens (23) are spheres, wherein the radius of curvature of the distal lens surface (26) of the corrective lens (23) is smaller in magnitude than the radius of curvature of the proximal lens surface (27) of the corrective lens (23).

**13.** The device according to any one of claims 1 to 12, **characterised in that** at least one of the elements corneal optics (18), holder (19) and ocular optics (20) is mounted on the carrier (17) so as to be adjustable along the central viewing axis (5) and/or transversely to the central viewing axis (5).

**14.** The device according to any one of claims 1 to 13, **characterised in that** the holder (19) is designed to interchangeably receive the intraocular lens (8).

**15.** Use of an ocular optics (20) comprising distally a corrective lens (23) and, proximal thereto, a diverging lens (24) and a converging lens (25), wherein the corrective lens (23) has a distal concave lens surface (26) and a proximal convex lens surface (27), and wherein preferably at least one of the mutually facing lens surfaces (28, 29) of the diverging and converging lenses (24, 25) is an asphere, in a device (16) for testing vision through an intraocular lens (8).

## Revendications

**1.** Dispositif pour contrôler la vue à travers une lentille intraoculaire (8), comprenant un support (17) qui supporte, dans un axe de vision central (5), se suivant de distal à proximal, une optique cornéenne (18), une fixation (19) pour la lentille intraoculaire (8) et une optique oculaire (20),

**caractérisé en ce que** l'optique oculaire (20) comprend une lentille de correction (23) distale et, de manière proximale à celle-ci, une lentille de dispersion (24) et une lentille convergente (25), dans lequel la lentille de correction (23) présente une surface de lentille concave distale (26) et une surface de lentille convexe proximale (27).

**2.** Dispositif selon la revendication 1, **caractérisé en ce qu'**au moins une des surfaces de lentille (28, 29) tournées l'une vers l'autre des lentilles de dispersion et de convergence (24, 25) est une asphère.

**3.** Dispositif selon la revendication 2, **caractérisé en ce qu'**au moins une des surfaces de lentille (28, 29) tournées l'une vers l'autre des lentilles de dispersion et de convergence (24, 25) est une asphère au moins du quatrième ordre.

**4.** Dispositif selon la revendication 3, **caractérisé en ce que** la hauteur de la flèche ($z_1$) mesurée à distance du plan de sommet (33) de l'asphère est choisie selon

$$z_1 = \frac{\rho \cdot r^2}{1+\sqrt{1-(p \cdot r)^2}} + A_2 \cdot r^2 + A_4 \cdot r^4 \text{ avec } A_4 < 0$$

$z_1$ hauteur de la flèche, mesurée de distal à proximal,
$\rho$ courbure du sommet,
r écart radial de l'axe de vision central (5), et
$A_2$, $A_4$ coefficients prédéfinis, dans lequel $A_2 > 0$, lorsque l'asphère est une surface de lentille de la lentille convergente (24), et $A_2 < 0$, lorsque l'asphère est une surface de lentille de la lentille de dispersion (25).

**5.** Dispositif selon l'une des revendications 2 à 4, **caractérisé en ce que** l'asphère est la surface de lentille distale (29) de la lentille convergente (25).

**6.** Dispositif selon l'une des revendications 2 à 5, **caractérisé en ce que** la surface de lentille (34) de la lentille convergente, respectivement de dispersion (25, 24), opposée à l'asphère est planaire.

**7.** Dispositif selon l'une des revendications 1 à 6, **caractérisé en ce qu'**au moins une des optiques cornéenne et oculaire (18, 20) présente un achromate (21, 22; 24, 25) ou un apochromate.

**8.** Dispositif selon l'une des revendications 1 à 7, **caractérisé en ce que** l'optique cornéenne (18) présente une lentille de dispersion cornéenne (21) et une lentille de convergence cornéenne (22).

9. Dispositif selon les revendications 7 et 8, **caractérisé en ce que** la lentille de dispersion cornéenne (21) et la lentille convergente cornéenne (22) présentent des nombres d'Abbe différents et/ou les lentilles convergentes et de dispersion (24, 25) de l'optique oculaire (20) présentent des nombres d'Abbe différents.

10. Dispositif selon la revendication 9, **caractérisé en ce que** la surface de lentille distale (36) de la lentille convergente cornéenne (22) est une asphère au moins du quatrième ordre.

11. Dispositif selon la revendication 10, **caractérisé en ce que** la hauteur de la flèche ($z_2$) de la surface de lentille distale mesurée à distance du plan de sommet (37) de cette surface de lentille distale (36) de la lentille de convergence cornéenne (22) est choisie selon

$$z_2 = \frac{\rho \cdot r^2}{1 + \sqrt{1 - (p \cdot r)^2}} + A_2 + r^2 + A_4 \cdot r^4 \text{ avec } A_2 > 0 \text{ et } A_4 < 0,$$

$z_2$ hauteur de la flèche, mesurée de distal à proximal,
$\rho$ courbure du sommet,
r écart radial de l'axe de vision central (5), et
$A_2$, $A_4$ coefficients prédéfinis.

12. Dispositif selon l'une des revendications 1 à 11, **caractérisé en ce que** la surface de lentille distale et proximale (26, 27) de la lentille de correction (23) sont des sphères, dans lequel le rayon de courbure de la surface de lentille distale (26) de la lentille de correction (23) a une valeur plus petite que le rayon de courbure de la surface de lentille proximale (27) de la lentille de correction (23).

13. Dispositif selon l'une des revendications 1 à 12, **caractérisé en ce qu'**au moins un des éléments optique cornéenne (18), fixation (19) et optique oculaire (20) le long de l'axe de vision central (5) et/ou transversalement à l'axe de vision central (5) est monté en pouvant être déplacé sur le support (17).

14. Dispositif selon l'une des revendications 1 à 13, **caractérisé en ce que** la fixation (19) est conçue pour recevoir la lentille intraoculaire (8) de manière interchangeable.

15. Utilisation d'une optique oculaire (20), qui comprend de manière distale une lentille de correction (23) et, de manière proximale à celle-ci, une lentille de dispersion (24) et une lentille convergente (25), dans lequel la lentille de correction (23) présente une surface de lentille concave distale (26) et une surface de lentille convexe proximale (27), et dans lequel de préférence au moins une des surfaces de lentille tournées l'une vers l'autre (28, 29) des lentilles de dispersion et de convergence (24, 25) est une asphère, dans un dispositif (16) pour contrôler la vue à travers une lentille intraoculaire (8).

*Fig. 1*

*Fig. 2*

*Fig. 3*

*Fig. 4*

*Fig. 5*

*Fig. 6*

*Fig. 7*

*Fig. 8*

**Fig. 9**

**Fig. 10**

**Fig. 11**

**Fig. 12**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2012068551 A